# FASCICULE DE BREVET EUROPEEN

(11) **EP 1 324 731 B1**
(45) Date de publication et mention de la délivrance du brevet: **07.12.2005**
(21) Numéro de dépôt: 01976413.3
(22) Date de dépôt: 12.10.2001
(51) Int. Cl.: A61F 9/013

(54) **DISPOSITIF DE CHIRURGIE CORNEENNE**
VORRICHTUNG FÜR DIE HORNHAUTCHIRURGIE
CORNEAL SURGERY DEVICE

(30) Priorité: 12.10.2000 FR 0013067
(43) Date de publication de la demande: 09.07.2003
(73) Titulaire: Moria SA, 92160 Antony (FR)
(72) Inventeur: AUFAURE, Jean-Luc, F-03210 SOUVIGNY (FR); SEMPE, Antoine, F-92130 ISSY LES MOULINEAUX (FR)
(74) Mandataire: Robert, Jean-Pierre
(86) Numéro de dépôt international: PCT/FR2001/003160
(87) Numéro de publication internationale: WO 2002/030344

(56) Documents cités:
- EP-A- 1 027 873
- WO-A-99/26568
- US-A- 4 423 728
- US-A- 5 980 543

## Description

La présente invention concerne un dispositif de chirurgie cornéenne, notamment un kératome capable de procéder à une découpe lamellaire de la cornée, totale ou partielle (c'est-à-dire pour former un volet cornéen).

Il existe de nombreux appareils aptes à réaliser cette opération (résection) cornéenne pour example l'appareil décrit dans le document EP 1 027 873. La technique de résection lamellaire a vu le jour il y a plus de cinquante ans et les appareils n'ont cessé d'évoluer depuis cette date. Cette évolution a porté essentiellement sur deux points : les moyens de découpe du disque cornéen et les moyens d'avancement des moyens de coupe de la cornée.

On recense aujourd'hui deux types principaux de moyens de coupe : les lames à mouvement circulaire (comme par exemple le microkératome du Professeur DRAEGER) et les lames à mouvement rectiligne alternatif, avec parmi celles-ci celles qui sont placées dans le plan de coupe (WO 95/31143) et celles, les plus employées, qui sont inclinées par rapport à ce plan à l'instar d'une lame de varlope (US 4 462 370 par exemple).

La source du mouvement de la lame est identique, à savoir un moteur ou une turbine dont l'arbre de sortie tournant est relié à la lame, soit sans modification de mouvement, la lame étant alors rotative, soit avec transformation du mouvement circulaire continu du moteur en un mouvement linéaire alternatif par le jeu d'un pion solidaire de l'arbre et d'une rainure solidaire de la lame qui est alors oscillante dans une direction perpendiculaire à la direction de son mouvement linéaire.

En ce qui concerne les moyens pour déplacer la lame de coupe au travers de la cornée à réséquer, ceux-ci ont en commun un anneau de fixation d'une base de l'appareil sur l'oeil qui porte des moyens de guidage coopérant avec une tête porte-lame à l'intérieur de laquelle le mouvement de la lame est entretenu. Ces moyens de guidage sont de deux types, soit des glissières (généralement rectilignes) dans lesquelles la tête porte-lame est montée coulissante (voir le document US 4 662 370), soit un pivot autour duquel la tête porte-lame est montée tournante (voir le document US 5 586 980).

Le mouvement de la tête porte-lame le long de ces moyens de guidage fixes est assuré par le chirurgien lui-même à la main. Il est limité par une butée dans le cas où la découpe cornéenne veut être limitée à la formation d'un volet encore relié à la cornée. Récemment, on a proposé de motoriser ce mouvement en faisant coopérer des moyens tournants sur eux-mêmes et portés par la tête porte-lame avec des moyens fixes portés par l'anneau de fixation de manière que les moyens tournants puissent rouler sans glisser sur les moyens fixes (voir à ce propos les documents US Re 35 421, US 5 624 456, US 5 980 543).

Les dispositifs actuels à avance motorisée sont, pour le chirurgien, des dispositifs compliqués à mettre en oeuvre. Il n'est en effet pas simple de régler le commencement et la fin de la trajectoire de coupe de la tête, et il n'existe aucune possibilité d'agir sur une vitesse de déplacement de la tête porte-lame car il n'existe qu'un seul moteur pour entraîner à la fois la lame dans son mouvement alternatif et les pignons d'avance du kératome par rapport à l'anneau avec un réducteur qui fixe la fréquence d'oscillation de la lame et la vitesse d'avance dans un rapport déterminé par construction. Enfin, ces kératomes ne peuvent pas être utilisés par des chirurgiens qui préfèrent commander manuellement l'avance du kératome au travers de l'anneau.

Par la présente invention on entend remédier à ces inconvénients avec un microkératome simple d'utilisation et qui offre l'avantage de répondre à tous les besoins exprimés par les chirurgiens.

A cet effet, l'invention a donc pour objet un dispositif de chirurgie cornéenne comprenant :
- une base annulaire de fixation de l'appareil sur l'oeil d'un patient,
- une tête de coupe comportant un corps et une lame et susceptible d'être déplacée selon une trajectoire plane parallèle à la base annulaire,
- des moyens de guidage de la tête de coupe par rapport à la base,
- des moyens d'entraînement de la lame dans la tête de coupe selon un mouvement linéaire alternatif parallèle à son fil de coupe,
- des moyens d'entraînement de la tête de coupe par rapport à la base annulaire selon la trajectoire susdite.

Selon l'invention, lesdits moyens d'entraînement comprennent deux ensembles moteurs indépendants surmontant la tête de coupe, et dont les arbres sont parallèles et perpendiculaires au plan de la trajectoire de la tête de coupe.

L'indépendance de ces moyens d'entraînement procure de nombreux avantages. Il est ainsi aisé au moyen d'une électronique simple d'asservir le fonctionnement de chacun des moyens à des paramètres fixes ou variables déterminés et à des conditions réalisant une relation entre l'un et l'autre.

Ainsi, il est possible pour le chirurgien d'ajuster la vitesse d'avance de la tête de coupe le long de sa trajectoire lui permettant ainsi d'ajuster cette vitesse à sa pratique. L'électronique de commande peut prévoir une vitesse variable d'avance de la tête de coupe en fonction par exemple de la variation de la surface de cornée en contact avec la lame. On peut également prévoir que l'oscillation soit coupée pendant la course de retour de la tête de coupe.

Il est également possible de prévoir des sécurités sur l'entraînement de l'avance de la tête à savoir neutralisation de l'avance si l'oscillation s'arrête ou présente une défaillance, impossibilité de mise en route en cas de défaillance du système d'aspiration de maintien de la base annulaire...

Le double entraînement selon l'invention rend possible de fixer avec précision et sans erreur possible pour le chirurgien, le point de départ de la trajectoire de manière que, à ce point, la lame soit toujours hors de contact avec la cornée et ce quel que soit le diamètre du capot cornéen que l'on souhaite obtenir.

On notera enfin que l'indépendance des moyens d'entraînement permet de supprimer tous les moyens mécaniques d'engrenages, de roue et vis sans fin qui sont nécessaires, et logés dans la tête pour dériver de l'arbre d'oscillation une transmission propre à assurer l'avance de la tête de coupe. Or cette transmission dérivée ne peut être réalisée qu'au moyen de pièces métalliques qui seules permettent d'obtenir la précision de fabrication requise pour ce matériel de petite dimension relevant de la micromécanique. Cette transmission réalise alors une continuité électrique entre le moteur du microkératome et l'anneau ou la base de fixation qui ne met pas à l'abri le patient d'une défaillance électrique du matériel pendant l'opération. L'invention, ayant supprimé cette transmission dérivée, peut mettre en oeuvre à la sortie du moteur d'entraînement de l'avance de la tête un arbre en matière diélectrique (matière plastique) qui forme une barrière isolante entre l'alimentation électrique du moteur et l'anneau (base) de fixation en contact avec l'oeil du patient.

D'autres caractéristiques et avantages de l'invention ressortiront de la description des exemples de réalisation donnés ci-après sans caractère limitatif.

Il sera fait référence aux dessins annexés parmi lesquels :
- la figure 1 est une représentation en coupe longitudinale d'un microkératome pivotant selon l'invention,
- la figure 2 est une vue extérieure de l'extrémité du carter commun aux deux moteurs par laquelle ce carter est accouplé à la tête de coupe,
- la figure 3 est une vue extérieure d'un exemple de réalisation du corps de la tête de coupe susceptible d'être adapté à l'extrémité du carter selon la figure 2,
- la figure 3A illustre par une vue latérale une tête de coupe conforme à la figure 3 équipée d'une lame de coupe,
- les figures 3B et 3C sont des vues partielles de dessus d'une lame utilisable dans un microkératome selon l'invention,
- la figure 4 est une vue extérieure d'une variante de réalisation du corps selon la figure 3,
- la figure 5 est une vue extérieure partielle du carter et de la tête de coupe assemblés avec un dispositif de verrouillage de cet ensemble sur la base annulaire (non représentée) de fixation de l'appareil sur l'oeil du patient,
- la figure 6 et la figure 7 illustrent par deux vues de dessous de la figure 5, les états inactif et actif du dispositif de verrouillage,
- la figure 8 est une vue de dessus simplifiée du dispositif selon l'invention illustrant deux points extrêmes de la trajectoire de la tête de coupe par rapport à la base annulaire du dispositif,
- la figure 9 illustre la tête de coupe accouplée à ses moyens d'entraînement, avec un organe de réglage du point terminal de la trajectoire de coupe,
- la figure 10 est une vue extérieure de la base annulaire de fixation du dispositif, avec des moyens de réglage du point terminal de la trajectoire de la tête de coupe,
- la figure 11 est une vue de dessous en perspective du dispositif selon l'invention dans lequel la base annulaire est une variante de réalisation de celle représentée à la figure 10, et la tête de coupe accouplée à ses moyens d'entraînement est celle représentée à la figure 5,
- la figure 12 est une vue semblable à la figure 1 d'un microkératome rectiligne selon l'invention,
- la figure 13 est une vue extérieure de côté de ce microkératome,
- la figure 14 est une vue extérieure de l'ensemble moteur et de la tête de coupe avant leur assemblage du microkératome rectiligne,
- la figure 15 est une vue extérieure de la base annulaire de ce microkératome.

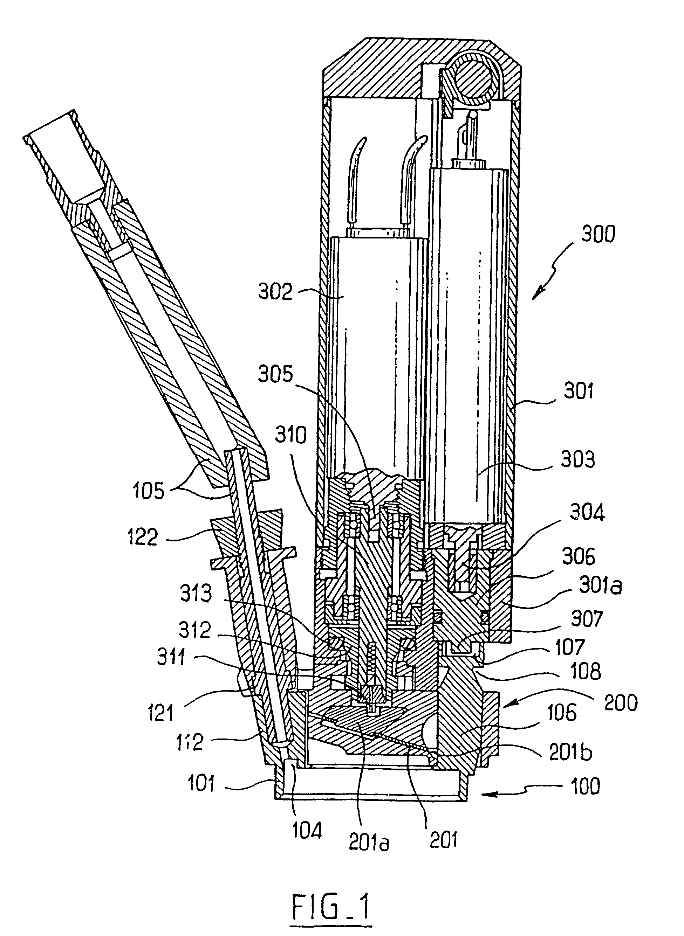

Le dispositif de chirurgie cornéenne représenté aux figures 1 à 11 comporte essentiellement trois parties. La première partie repérée 100 sur les figures forme une base annulaire de fixation du dispositif sur l'oeil d'un patient. La deuxième partie repérée 200 sur les figures, constitue une tête de coupe pour, au moyen d'une lame 201 qu'elle comporte, découper un disque ou un volet cornéen. La troisième partie 300 de l'appareil selon l'invention est constituée par les moyens d'entraînement d'une part de la tête de coupe 200 et d'autre part de la lame de coupe 201 à l'intérieur de la tête de coupe.

La base annulaire 100, représentée notamment aux figures 1, 8, 10 et 11 comprend un anneau 101 formé par une couronne annulaire 102 équipée d'une jupe périphérique 103. De manière connue, la périphérie interne de la couronne annulaire 102 et le bord inférieur de la jupe 103 viennent en contact étanche avec l'oeil du patient et délimitent avec ce dernier une chambre annulaire intérieure qui est connectée à une source d'aspiration par un orifice 104 et un tube disposé à l'intérieur d'un manche 105. Ainsi la cornée à opérer fait-elle saillie à l'intérieur de l'anneau 101.

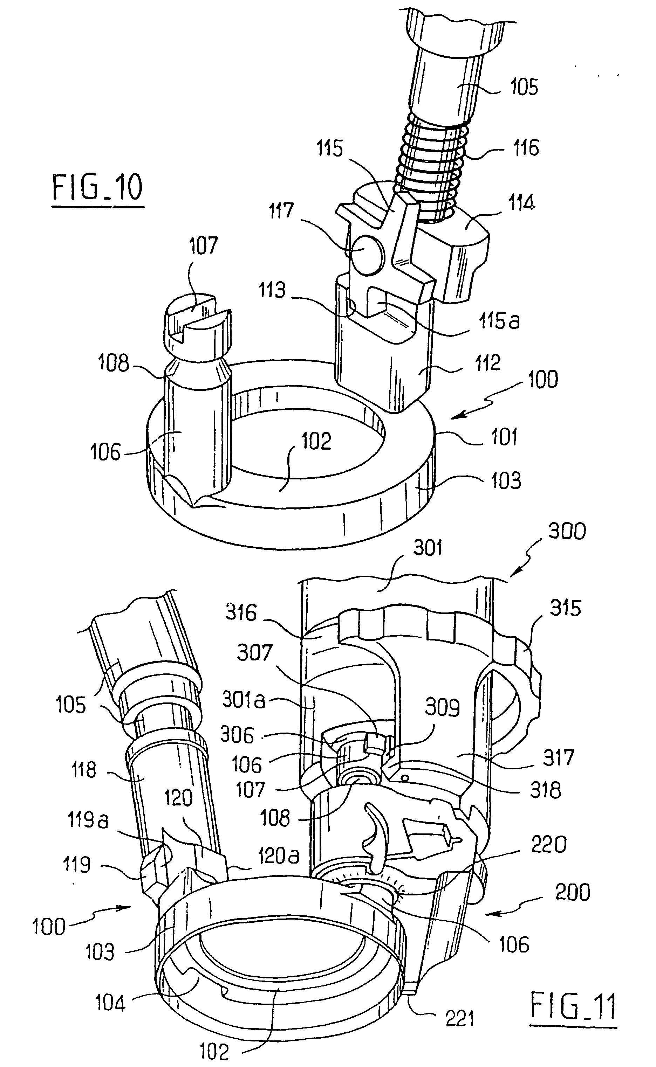

Cet anneau possède, d'une seule pièce avec lui, un pivot 106 qui est perpendiculaire au plan de la couronne annulaire 102. L'extrémité supérieure de ce pivot 106 est pourvue de reliefs qui sont ici réalisés sous la forme d'une mortaise 1.07. Aux figures 1, 10 et 11, cette extrémité supérieure possède, sous la mortaise 107, une gorge 108. A la figure 8, le pivot représenté possède en partie supérieure une surface latérale dont une première zone 109 est de diamètre inférieur à une seconde zone 110, la zone 110 étant quant à elle équipée d'une gorge 111 dont le fond est au diamètre de la zone 109.

Le pivot 106 est situé dans une zone de l'anneau 101 sensiblement diamétralement opposée au manche 105. Le manche 105 est connecté à l'anneau 101 par une pièce de base 112 qui offre une surface de butée 113 sensiblement radiale. Dans le cas de la figure 10, le manche 105 comporte une pièce coulissante 114 équipée d'un jeu de cales 115 formé par une pièce en étoile dont les branches sont d'épaisseurs différentes. La pièce 114 est appliquée sur la pièce 112 au moyen d'un ressort 116. On comprend que, lorsqu'on soulève la pièce 114 à l'encontre du ressort 116 le long du manche 105, on dégage les branches de la pièce en étoile 115 de la butée 113, permettant ainsi de choisir la branche d'épaisseur appropriée pour venir en butée contre la surface 113. Chacune des branches offre alors une surface 115a en avant de la surface 113 et distante de cette dernière d'une valeur égale à l'épaisseur de la branche. On notera que la pièce 115 est montée à rotation autour d'un axe 117 porté par la pièce 114.

Dans le cas de la figure 11 et de la figure 1, le manche 105 possède un manchon 118 extérieur, équipé à sa base de plusieurs, par exemple trois, protubérances 119, 120. Ce manchon peut être indexé angulairement autour du manche 105 de sorte que l'une des protubérances 119, 120, présente une surface de butée 119a ou 120a similaire à la surface de butée 113 ou à celle 115a de la figure 10. Les protubérances 119 et 120 sont de dimensions circonférentielles différentes si bien que les surfaces 119a et 120a forment des butées qui sont plus ou moins proches angulairement du pivot 106 selon la dimension de la protubérance. L'indexation de ce manchon 118 autour du manche 105 peut s'opérer au moyen d'une partie cannelée 121 de ce manche coiffée par une partie cannelée en correspondance du manchon 118, un écrou 122 se vissant sur le manche 105 permettant d'appliquer le manchon 118 sur la pièce d'implantation du manche 112, en prise avec les cannelures 121 ou, lorsqu'il est dévissé, de faire glisser ce manchon 118 pour changer la protubérance qui doit être en service. Ce n'est pas sortir de l'invention que de prévoir comme à la figure 10, un ressort en tête du manchon 118 aux lieu et place de l'écrou 122.

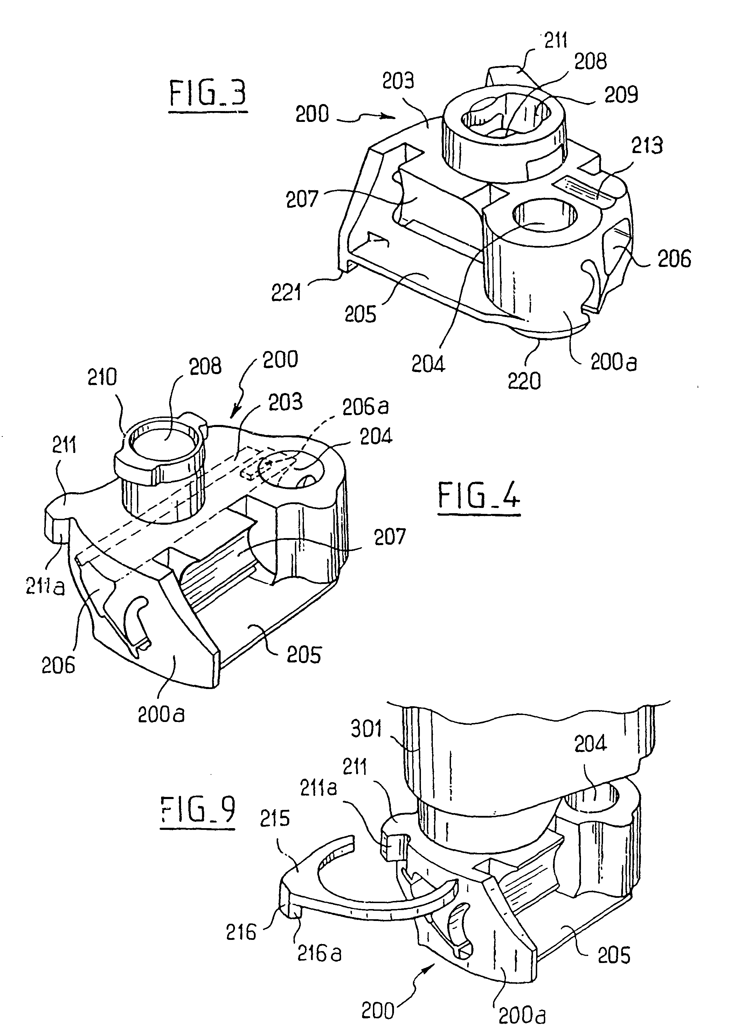

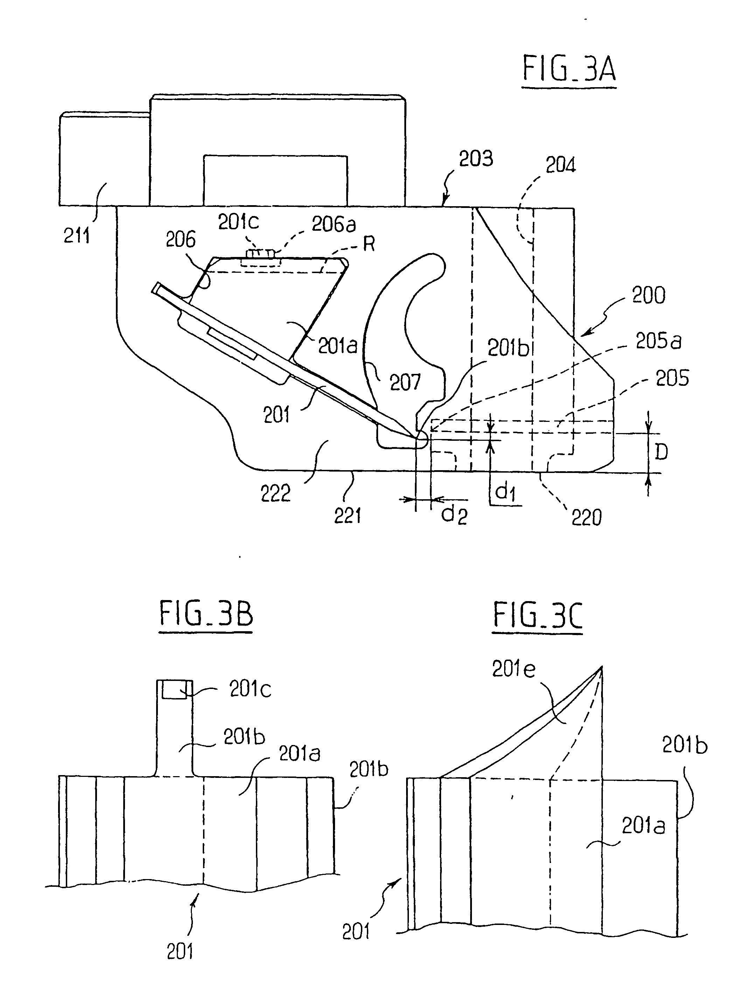

La tête de coupe 200 visible aux figures 1, 3, 3A, 4, 5, 8, 9 et 11, est d'une géométrie générale déjà décrite dans l'état de la technique. Cette tête de coupe possède un corps monobloc 200a comportant une surface supérieure 203. Une surface inférieure est celle qui sera tournée vers l'anneau de fixation 101 pour y glisser. Elle est dans le cas des figures réduite à une surface 220 et une surface 221, toutes deux dans un plan commun. Ce corps possède tout d'abord un orifice traversant 204 débouchant dans les surfaces 220 et 203 qui est destiné à recevoir le pivot 106. Ce corps définit en outre, perpendiculairement à l'axe de cet orifice 204, un plateau 205 dont la face inférieure est distante des surfaces 220 et 221 et dont le rôle est d'aplanir la cornée juste devant la lame de coupe. Le corps 200a possède également, à la suite du plateau, un orifice 206 de logement et de guidage de la lame de coupe 201 représentée aux figures 1, 3A, 3B, 3C. Cette lame de coupe 201 possède un support 201a dans la surface supérieure duquel est ménagée une rainure R perpendiculaire au fil 201b de la lame, lequel est sensiblement parallèle au bord arrière du plateau 205, cette rainure étant destinée à recevoir un doigt excentré de l'arbre de sortie du moteur d'entraînement de la lame. Le logement 206 débouche immédiatement derrière le plateau 205, pour permettre à la lame de déborder et à son fil 201b d'être situé en-dessous de la face inférieure du plateau 205 et en arrière de son bord arrière 205a à une distance calibrée (d1, d2) qui est en relation avec l'épaisseur du volet cornéen à réaliser. Le corps 200a présente au-dessus de cette ouverture, et juste à l'arrière du plateau 205, une surface déflectrice 207, elle-même aussi bien connue de l'état de la technique, qui forme surface de glissement pour le volet cornéen au fur et à mesure de sa découpe.

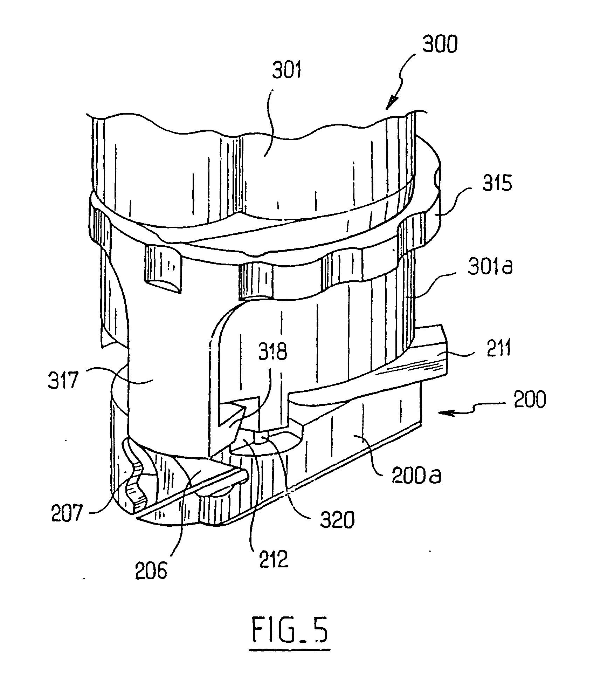

Un orifice 208, parallèle à l'orifice 204, débouche à l'intérieur du logement 206 à partir de la surface supérieure 203 du corps. A la figure 3 on remarque que l'orifice 208 est prolongé par des moyens 209, du genre moyens femelles d'accouplement à baïonnette, qui permettent d'atteler la tête à l'extrémité de moyens 300 d'entraînement du kératome. A la figure 4, ces moyens à baïonnette sont représentés sous forme d'un manchon 210 dont les ergots pénétreront et coopéreront avec des moyens femelles prévus dans les moyens d'entraînement 300. Cet orifice 208 permet au doigt d'entraînement de la lame d'atteindre la rainure R que comporte son support 201a.

On remarquera que le corps 200a comporte, au voisinage de sa surface supérieure 203, une excroissance latérale 211 qui présente une surface 211a sensiblement parallèle à l'axe de l'orifice 204, cette surface étant destinée à former un moyen de butée pour coopérer avec l'une des surfaces 113, 115a, 119a ou 120a prévues sur la base annulaire du dispositif.

Le logement 206 pour la lame 201 et son porte-lame 201a peut soit traverser de part en part le corps 200a de la tête de coupe 200, soit être limité par une cloison terminale. Cette cloison pourrait être un voile en continuité de la surface latérale de la tête représentée à la figure 3 au voisinage de l'alésage 204.

Dans le cas d'un orifice traversant, principalement prévu dans une tête à usage multiple, réalisée en métal, il convient d'empêcher l'ensemble lame 201 porte-lame 201a de s'échapper de son logement alors qu'il y a été introduit et que la tête n'est pas encore montée sur l'ensemble moteur 300, au cours des manipulations nécessaires à ce montage par le chirurgien. Pour ce faire, le logement 206 possède une petite cavité 206a pour le logement d'un ergot 201c porté à l'extrémité d'une languette élastique 201d qui est en une seule pièce avec le support 201a de la lame 201 (voir figures 3A, 3B et 4). Lors de l'introduction de la lame dans la cavité 206 la languette 201d est élastiquement courbée jusqu'à ce que l'ergot 201c se relève et se loge dans le logement 206. La lame est alors retenue dans le logement 206. Elle peut néanmoins osciller dans ce logement 206 sous l'effet du doigt excentré d'entraînement car la longueur de la cavité 206a mesurée dans la direction de l'oscillation est supérieure à l'amplitude de cette oscillation. La lame est en outre maintenue dans le logement dans une position telle que la rainure R du support est maintenue dans une région de la tête qui sera nécessairement balayée par le doigt excentré d'entraînement dès la première rotation du moteur, voire dès le montage de la tête sur le bloc 300 d'entraînement. A cet égard, il sera avantageux que la rainure R s'élargisse en partie supérieure de sorte que la pénétration du doigt d'entraînement en sera facilitée.

Dans le cas où le logement 206 est fermé par un voile d'extrémité, c'est principalement le cas où la tête 200 est à usage unique en matière plastique, elle doit être livrée équipée de sa lame. L'ergot 201c sert alors d'organe de maintien de la lame dans la tête 200 à usage unique depuis sa fabrication jusqu'à son utilisation.

Dans une variante de réalisation de la lame 201, représentée à la figure 3C, le support 201a est pourvu d'une pointe saillante 201e au-delà d'un bord de la lame 201 qui sert de guide à l'introduction de la lame dans le logement 206.

Lorsque la tête de coupe 200 est montée pivotante autour du pivot 106, sa trajectoire est plane, perpendiculaire à l'axe de ce pivot 106 et donc parallèle à l'anneau 101.

Les moyens d'entraînement 300 comportent, dans un carter 301, deux groupes moteurs 302, 303 dont les carcasses sont solidaires en rotation du carter, et qui présentent des arbres de sortie respectivement 304 et 305. Sur l'arbre 304 de sortie du groupe moteur 303 on a calé en rotation un arbre prolongateur 306 dont l'extrémité 307 est au voisinage de l'extrémité 301a du carter. L'extrémité 307 de cet arbre prolongateur 306 est en forme de tenon dont les dimensions sont adaptées à une pénétration sans jeu dans la mortaise 107 située en tête du pivot 106.

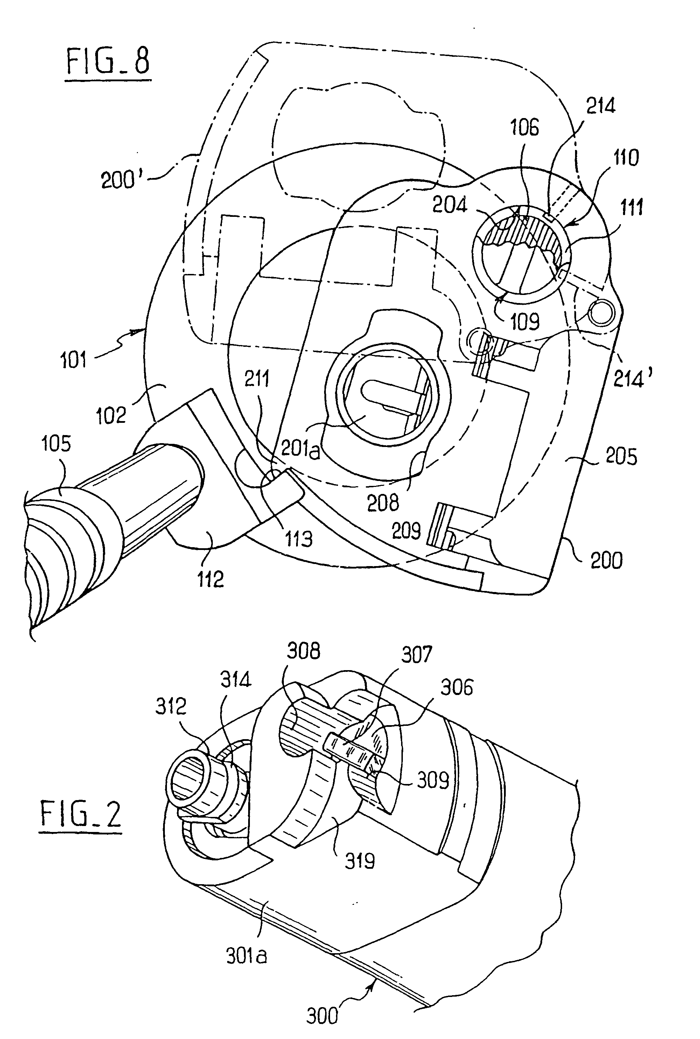

On notera, au vu de la figure 2, que l'extrémité 301a du carter 301 est une pièce usinée relativement complexe qui présente, dans le prolongement de l'arbre 306, un évidement partiellement cylindrique 308, coaxial à l'arbre 306 mais de plus petit diamètre que ce dernier, de sorte qu'un des bords 309 de cet évidement forme une butée angulaire pour le tenon 307. En effet le tenon 307 n'a pas pour longueur le diamètre de l'arbre 306, mais une longueur inférieure pour que l'une de ses extrémités puisse librement tourner dans l'évidement 308, tandis que son autre extrémité soit contrainte de buter sur le bord 309 de ce dernier. On comprend qu'ainsi ce bord 309 constitue une butée d'indexation angulaire du tenon 307 par rapport au carter 301.

L'arbre de sortie 305 du moteur 302 est également prolongé par un arbre 310 qui comporte à son extrémité un doigt excentré 311 d'entraînement de la lame 201. Cet arbre 310 est entouré à son extrémité d'un manchon 312 (seul le manchon 312 est représenté à la figure 2, l'arbre 310 ayant été omis pour des raisons de clarté du dessin), ce manchon 312 étant fixe en rotation par rapport à la pièce terminale 301a du carter 301, mais étant susceptible d'un léger déplacement axial le long de l'arbre 310 à l'encontre d'un ressort annulaire en caoutchouc 313 qui tend à le faire légèrement rentrer à l'intérieur de la pièce d'extrémité 301a. Ce manchon 312 est pourvu d'une collerette 314 dont la forme, qui n'est pas de révolution, constitue des moyens mâles d'accouplement à baïonnette avec les moyens femelles 209 du corps 200a de la tête de coupe 200 telle que représentée à la figure 3.

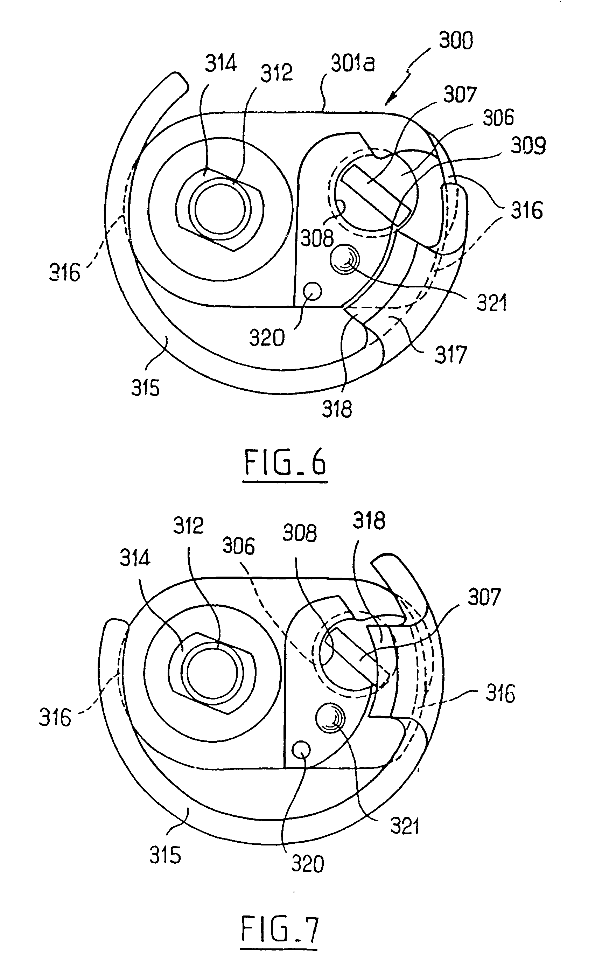

Aux figures 5, 6 et 7 on a représenté les éléments 300 et 301a équipés d'un verrou formé par une bague partielle 315 montée tournante dans une gorge 316 de la pièce 301a. Cette bague 315 présente un bras inférieur 317 muni d'une dent tournée vers l'intérieur 318, cette dent s'étendant à un niveau légèrement écarté du tenon 307 vers l'extrémité libre de la pièce 301a. Comme cela est visible aux figures 6 et 7, lorsque la bague 315 est dans un état inactif (figure 6), la dent 318 se trouve logée dans un évidement 319 de la pièce d'extrémité 301a. Lorsqu'elle est en état de service (figure 7), la dent 318 vient interférer avec l'évidement 308 pour ainsi, lorsque le pivot sera logé dans cet évidement, pénétrer dans la gorge 108 de ce pivot et interdire toute séparation axiale entre la pièce 301a, qui portera bien entendu la tête de coupe 200 et la base annulaire 100.

En vue d'une opération, le chirurgien commence par assembler la tête de coupe 200, préalablement équipée de la lame de coupe 201 à l'ensemble 300 des moyens moteurs. Pour ce faire, il procède à l'emboîtement des moyens 209 de la tête 200 et des moyens 314 de l'extrémité 301a de l'ensemble 300. L'orientation angulaire fixe des moyens 314 est telle que cet emboîtement ne peut être réalisé que si la tête est décalée angulairement par rapport à la pièce 301a, c'est-à-dire si les axes du logement 204 et de l'arbre 306 ne sont pas en correspondance. L'emboîtement axial réalisé, le chirurgien fait pivoter la tête 200 autour de l'axe de l'arbre 310 logé à l'intérieur des moyens 314 pour que ceux-ci coopèrent comme un accouplement à baïonnette avec les moyens 209, empêchant ainsi toute séparation axiale de la tête 200 du carter 301. Cette rotation se fait dans le sens du rapprochement de l'axe de l'orifice 204 avec l'axe de l'arbre 306. La coaxialité de ces axes est obtenue lorsque par exemple, une butée (un pion) 320 prend appui sur une surface 212 du corps 201 de tête de coupe (voir figure 5). On aura par ailleurs prévu avantageusement un indexage de la tête sur le carter au moyen par exemple d'une goujure 213 (figure 3) ménagée à la surface supérieure 203 de la tête qui vient recevoir une bille 321 (figure 6 et figure 7) faisant saillie au moyen d'un ressort de rappel de la face terminale de la pièce 301.

On comprend que le montage de la tête 200 de la figure 4 sur le corps de carter 301 se réalise de la même manière, seuls les moyens d'accouplement à baïonnette mâles et femelles étant inversés par rapport à l'exemple précédent. On notera à cet égard que les moyens 210 qui réalisent cet accouplement à baïonnette ne sont pas symétriques, et constituent un détrompeur pour l'assemblage de la tête au carter de l'ensemble moteur interdisant de réaliser une fausse manoeuvre. Une telle dissymétrie dans ces moyens d'accouplement peut être également mise en oeuvre dans l'exemple de réalisation précédent.

Les éléments 200 et 300 étant ainsi assemblés et calés axialement et angulairement l'un sur l'autre, l'orifice 204 de la tête 200 se trouve dans l'alignement de l'arbre 306. On notera que l'arbre 306 possède des moyens de sa commande tels que, après une opération, il est ramené dans une position angulaire fixe qui est celle représentée à la figure 2, c'est-à-dire que le tenon 307 est en appui sur la surface 309 de la pièce 301a. Le chirurgien place la base annulaire 100 sur l'oeil du patient et l'y maintient par aspiration comme décrit précédemment. Il présente ensuite les éléments 200, 300 assemblés de manière à introduire le pivot 106 dans l'orifice 204. Pour obtenir que le tenon 307 pénètre dans la mortaise 107, le chirurgien doit faire pivoter l'ensemble des éléments 200, 300 autour du pivot 106, ce qui détermine une position angulaire relative et une seule entre la base 100 et l'ensemble des éléments 200, 300. Cette position est la position de départ de l'opération. Elle est atteinte alors que la lame de coupe est distante de la cornée à opérer du fait même que le sommet du tenon 307 repose sur le sommet du pivot 106 tant que le tenon n'a pas pénétré dans la mortaise. Il s'agit là d'une sécurité qui empêche toute blessure intempestive de la cornée par la lame de coupe. Lorsque le tenon 307 a pénétré dans la mortaise 107 l'ensemble moteur 300 / tête de coupe 200 repose sur la base annulaire 100 par les deux surfaces coplanaires 220 entourant l'orifice 204 et 221 d'extrémité de la paroi latérale 222 du corps 200a de la tête de coupe 200 (voir figures 3 et 3A). On comprend qu'en faisant varier la distance verticale D (figure 3A) qui sépare la face inférieure du plateau 205 de ces surfaces 220, 221 on fait varier l'écrasement de la partie de cornée faisant saillie au centre de la base annulaire 100 par le plateau 205. Ainsi plus cette distance est faible et plus la cornée est aplatie ; cela se traduit par l'obtention d'un capot cornéen de diamètre plus important que pour une valeur de D plus importante. En fournissant, toutes choses égales par ailleurs, un jeu de têtes de coupe 200 possédant chacune une valeur de la distance D différente, on offre au chirurgien la possibilité de choisir le diamètre du capot cornéen qu'il souhaite obtenir, le mieux adapté au sujet qu'il traite.

La trajectoire de la lame de coupe est donc une trajectoire circulaire centrée sur l'axe du pivot 106 et dans un plan perpendiculaire à l'axe de ce pivot. Par construction, les axes des moteurs 302 et 303 sont alors parallèles à l'axe du pivot 106 et donc perpendiculaires à la trajectoire de la lame de coupe. On notera qu'ainsi il n'existe pratiquement aucun porte-à-faux de l'ensemble mobile par rapport à la base annulaire fixe durant toute la procédure de découpe de la cornée. En effet, le poids du moteur 303 s'exerce pratiquement dans l'axe du pivot, et le poids du moteur 302 s'exerce le long d'un axe qui ne dépasse jamais la jupe périphérique 103 de la base annulaire. La résultante de ces deux forces de gravité est donc toujours à l'intérieur de la base annulaire.

Pour réaliser une liaison de sécurité axiale entre la base annulaire et l'ensemble mobile 200, 300, on a prévu selon l'invention plusieurs dispositifs.

Il s'agit en premier lieu du verrou représenté aux figures 5, 6, 7 et 11. Lorsqu'il est dans son état des figures 6 et 11, ce verrou n'interfère pas avec la tête de pivot qui a traversé l'orifice 204 et qui est en prise avec le tenon 307. En faisant tourner la bague 315 pour mettre le verrou dans son état actif, on fait passer la dent 318 sous la tête du pivot 106 pour le loger dans la gorge 108 de ce pivot. Il est alors impossible de séparer l'ensemble mobile 200, 300 de la bague 100, ou que cet ensemble mobile se soulève accidentellement au cours d'une opération. On n'a pas représenté aux figures les dispositifs d'indexation et de butée qui sont prévus entre la bague 315 et la pièce 301a, permettant de rendre sensibles au chirurgien les deux positions du verrou et d'éviter que ce verrou ne soit forcé dans un sens préjudiciable à la qualité mécanique du produit. Ces dispositifs peuvent tout simplement être des systèmes d'encliquetage élastique et des pions venant en butée sur des surfaces appropriées.

Cette fonction de verrouillage peut être réalisée autrement, comme représenté schématiquement à la figure 8. Sur cette figure on a représenté en trait plein la tête 200 à la fin de sa course de travail, et en trait mixte 200' cette tête au moment où le tenon 307 pénètre dans la mortaise 107. Le corps de tête 200a porte un ergot 214 qui fait saillie à l'intérieur de l'orifice 204 dans une position angulaire bien déterminée. Au moment où le tenon 307 pénètre dans la mortaise 107, cet ergot 214 est situé en face de la zone latérale 109 du pivot 106 qui est de diamètre plus petit que le diamètre intérieur de l'orifice 204, ce qui permet à la partie saillante de l'ergot 214 de ne pas empêcher le coulissement axial de la tête sur le pivot 106. Cet ergot est alors dans sa position 214', c'est-à-dire juste à l'entrée de la gorge 111 que porte la zone 110 du pivot qui, elle, est de diamètre sensiblement égal au diamètre intérieur de l'orifice 204. On comprend que, dès qu'on amorce le pivotement de la tête 200 autour du pivot 106 pour la faire passer de sa position 200' à sa position en trait plein sur la figure 8, l'ergot 214 s'engage dans la gorge 111. Pendant toute la durée de la coupe et le retour de la partie mobile autour du pivot 106, aucune désolidarisation axiale n'est possible entre la partie mobile 200, 300 et la base annulaire fixe. Dans le cas d'un microkératome susceptible d'être équipé au choix du chirurgien par des têtes qui diffèrent l'une de l'autre par la valeur D indiquée à la figure 3A on a, selon l'invention, prévu des moyens de liaison de sécurité axiale qui prennent en compte de manière simple cette différence de géométrie.

Dans une première variante non représentée la base annulaire 100 possède un pivot 106 démontable (par exemple du fait d'une implantation par vissage dans l'anneau) qui ainsi peut être apparié à la tête utilisée. En effet pour une tête à D faible, le tenon 307 et la dent 318 de la bague 315 sont plus proches de la surface supérieure de l'anneau 101. Le pivot correspondant à implanter sur l'anneau doit donc être plus court que pour une tête à D plus grand afin que le tenon 307 ne bute pas sur le fond de la mortaise 107 et que la gorge 108 ne soit pas trop haute par rapport à la dent 318.

Dans une autre variante, on peut prévoir une base 100 unique pour toutes les têtes en ayant réalisé une mortaise 107 suffisamment profonde pour accueillir un tenon 307 suffisamment long pour absorber les différentes hauteurs des têtes de coupe du fait des différentes dimensions D. Pour l'ajustement du mécanisme de liaison, il est alors prévu un bras 317 de longueur ajustable au moyen de deux parties télescopiques et d'un système d'indexage de l'ajustement faisant correspondre la longueur du bras à la tête choisie.

Dans une troisième variante, le kératome selon l'invention comporte un jeu de tête 200 avec pour chacune une distance D déterminée en fonction du diamètre du capot cornéen à obtenir et un jeu de bases annulaires 100 complet dont chacune correspond à une tête. Ces dispositions sont avantageuses notamment dans le cas où on prévoit des têtes de coupe et des bases à usage unique en matière synthétique.

On a vu que le point de départ de la trajectoire de la tête de coupe était fixé par l'orientation angulaire des tenons dans la pièce 301a. Le chirurgien peut alors commander l'alimentation du moteur 302 qui provoque le mouvement alternatif à haute fréquence de la lame 201. Il commande ensuite l'alimentation du moteur 303. Comme l'axe 306 lié à ce moteur ne peut pas tourner à cause de la liaison tenon - mortaise entre cet arbre 306 et le pivot fixe 106, c'est la carcasse de ce moteur qui va tourner autour de l'arbre 306, et donc l'ensemble du carter 301 et la tête de coupe 200 qui lui est associée. La rotation de cet ensemble mobile est arrêtée lorsque la saillie latérale 211 solidaire de la tête de coupe 200 vient en butée contre l'une des surfaces 113, 115a, 119a ou 120a, selon les modes de réalisation représentés, qui elles sont portées par une pièce solidaire de la base annulaire 100. Par un détecteur approprié sensible par exemple à l'augmentation du courant d'alimentation du moteur 303, l'alimentation de ce moteur est coupée, voire inversée, de manière à provoquer le retour de la tête à sa position initiale. On rappellera que cette position initiale est obtenue par l'arrivée en butée du tenon 307 contre une surface 309 portée par la pièce 301a. Ici aussi par un capteur approprié, on détecte l'augmentation brutale du couple résistant au mouvement de rotation et l'alimentation du moteur 303 est coupée. Les parties 200, 300 peuvent alors être retirées de la base annulaire 101, et on peut procéder à toute ablation intra-stromale en ayant maintenu le capot cornéen relevé, notamment par une procédure connue sous le nom de LASIK.

On comprend que la position finale de la tête de coupe détermine la position, la longueur et donc les qualités mécaniques de la zone de liaison dite charnière entre le volet qui vient d'être découpé et l'oeil. Le chirurgien doit pouvoir agir sur cette position finale. Il doit donc pouvoir déplacer la surface de butée 113 solidaire de la base annulaire 100. L'invention propose à cet effet plusieurs variantes de réalisation pour obtenir ce résultat, illustrées respectivement aux figures 10, 11 et 9.

On rappellera que le chirurgien peut, grâce au dispositif de la figure 10 déjà décrit, substituer à la surface 113 de butée une surface 115a choisie parmi quatre à sa disposition qui lui permettent de régler la position du point terminal de la trajectoire de la tête de coupe.

A la figure 11, ce sont les surfaces 119a et 120a qui peuvent se mettre en service sélectivement par rotation du manchon 118 qui les porte pour obtenir le même résultat.

A la figure 9 enfin, on propose une troisième variante de réalisation dans laquelle on agit non plus sur la surface 113 solidaire de la base annulaire 100 mais sur la surface 211a solidaire de l'excroissance 211 portée par le corps 200a de la tête de coupe. Lorsque la tête 200 est assemblée au carter 301, ce dernier présente une surface extérieure apte à recevoir une sorte de clip 215 qui possède une cale 216 pouvant prendre appui sur la surface 211a de l'excroissance 211 et présenter ainsi une surface 216a de butée venant coopérer avec la surface 113 de la pièce 112 de la base 100. Le chirurgien disposera d'un jeu de clips 215 pour pouvoir bénéficier de cales 216 d'épaisseurs différentes suffisamment variées pour qu'il puisse obtenir le réglage qui lui convient.

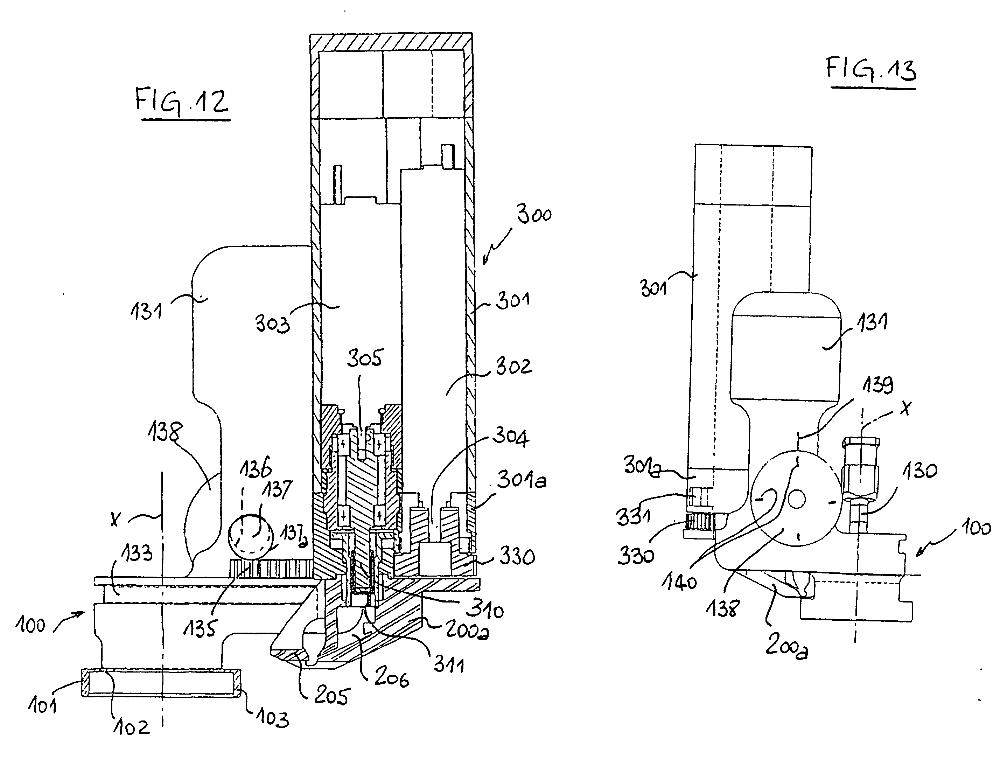

La description donnée ci-dessus en regard des figures 1 à 11 concerne un microkératome pivotant. Les figures 12 à 15 illustrent un microkératome à trajectoire rectiligne qui reprend toutes les caractéristiques déjà décrites à l'exception de celles relatives aux moyens de guidage et d'avancement pivotant remplacés par des moyens de guidage et d'avancement rectiligne. On retrouve sur ces figures des éléments déjà décrits avec les mêmes références et notamment une première partie 100 formant la base annulaire de fixation de l'appareil sur l'oeil, une seconde partie 200 constituant une tête de coupe et une troisième partie 300 qui comprend les moyens moteurs fournissant à l'appareil l'énergie mécanique nécessaire à l'entraînement de la tête de coupe par rapport à la base annulaire et au mouvement de la lame de coupe à l'intérieur de la tête de coupe.

Comme dans le cas des figures 1 à 11, la base annulaire 100 comprend un anneau 101 formé par une couronne annulaire 102 équipée d'une jupe périphérique 103. De manière connue, la périphérie interne de la couronne annulaire 102 et le bord inférieur de la jupe 103 viennent en contact étanche avec l'oeil du patient et délimitent avec ce dernier une chambre annulaire intérieure qui est connectée à une source d'aspiration par un orifice non représenté et un embout de connexion 130. Cet anneau est surmonté par deux bras supérieurs 131 et 132 placés à l'arrière de l'axe X de l'anneau 101 et sensiblement parallèles à cet axe qui forment un préhenseur de l'anneau par le chirurgien. La racine de ces bras, par laquelle ils sont reliés à l'anneau, comporte deux rainures parallèles 133, 134 qui, de section carrée, définissant une glissière parallèle au plan supérieur de l'anneau 101 dans l'ouverture centrale duquel la cornée du patient fait saillie. Au-dessus de l'une 133 de ces rainures, la surface intérieure de la racine du bras 131 porte une crémaillère rectiligne 135 dont le rôle sera décrit ci-après. On notera enfin la présence d'un orifice 136 au-dessus de la crémaillère pour la fixation d'un disque 137 sur la face interne du bras 131 dont le contour 137a est excentré par rapport à l'orifice 136 pour constituer une butée réglable au mouvement de la tête par rapport à la base comme décrit ci-après. Un bouton de manoeuvre 138 de ce disque, extérieur au bras 131 permet à l'aide d'un repère 139 et d'index 140 de fixer la position de la butée par rapport à la base.

La tête de coupe 200 visible aux figures 12, 13 et 14, est comme celle décrite précédemment d'une géométrie générale déjà décrite dans l'état de la technique. Cette tête de coupe possède un corps monobloc 200a comportant une surface supérieure 203. Une surface inférieure est celle qui sera tournée vers l'anneau de fixation 101. Ce corps définit en outre un plateau 205 dont le rôle est d'aplanir la cornée juste devant la lame de coupe. Le corps 200a possède également, à la suite du plateau, un orifice 206 de logement et de guidage d'une lame de coupe non représentée mais semblable à celle précédemment décrite.

Un orifice 208 débouche à l'intérieur du logement 206 à partir de la surface supérieure 203 du corps. L'orifice 208 comporte comme dans le cas de la figure 3 des moyens 209 femelles internes d'accouplement à baïonnette, qui permettent d'atteler la tête à l'extrémité de moyens 300 d'entraînement du kératome. Cet orifice 208 permet au doigt d'entraînement de la lame d'atteindre la rainure que comporte son support comme précédemment décrit.

On remarquera que le corps 200a comporte, au voisinage de sa surface supérieure 203, une excroissance latérale 230 destinée à former un moyen de butée pour coopérer avec les moyens d'entraînement 300.

Enfin le corps 200a possède au voisinage de sa surface supérieure 203, deux nervures latérales 231, 232 dont la direction est sensiblement parallèle au plateau 205 et dont la section carrée est identique à celle des rainures correspondantes 133, 134 de la base 100 qui formeront les glissières de guidage du déplacement rectiligne de la tête par rapport à la base.

Comme dans l'exemple de réalisation précédent, les moyens d'entraînement 300 comportent, dans un carter 301, deux groupes moteurs 302, 303 dont les carcasses sont solidaires en rotation du carter, et qui présentent des arbres de sortie respectivement 304 et 305.

L'arbre de sortie 305 du moteur 302 est prolongé par un arbre 310 qui comporte à son extrémité un doigt excentré 311 d'entraînement de la lame. Cet arbre 310 est entouré à son extrémité d'un manchon 312 qui, comme déjà décrit, constitue des moyens mâles d'accouplement à baïonnette avec les moyens femelles 209 du corps 200a de la tête de coupe 200.

L'arbre de sortie 304 du groupe moteur 303, contrairement à l'exemple précédent, porte un pignon d'extrémité 330 qui fait saillie latéralement de l'extrémité 301a du carter 301. L'extrémité axiale de ce pignon 330 est située sensiblement dans le plan d'extrémité de la partie 301a du carter 301. Par ailleurs, au-dessus de ce pignon, le carter 301 porte un doigt latéral 331 qui, le microkératome en service, viendra coopérer avec le disque butée 137 de la base 100 pour arrêter le déplacement de la tête au-dessus de l'anneau 101.

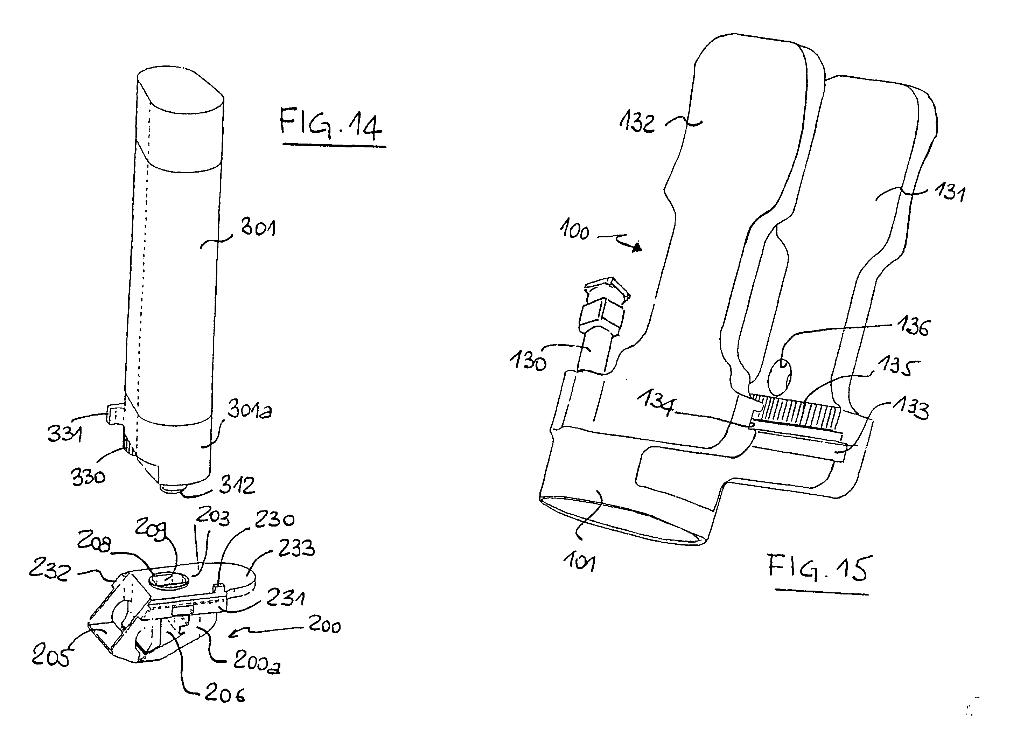

A la figure 14 on a représenté les ensembles 200 et 300 séparés. Leur accouplement consiste à introduire le manchon 312 dans l'orifice 208 de la tête et de procéder à une rotation relative des deux ensembles dans le sens qui amène le pignon 330 au-dessus d'un plateau arrière 233 de la tête venant masquer le flanc inférieur libre du pignon 330. La coopération des moyens 209 d'accouplement de la tête au manchon 212 est pleinement réalisée lorsque le flanc latéral de l'ensemble 300 vient en butée contre l'excroissance 230 de la tête en fin de rotation d'assemblage. On peut prévoir, comme dans l'exemple précédent, un indexage entre tête et bloc moteur pour fixer cette position.

Une fois cet assemblage réalisé, le chirurgien le présente entre les bras 131, 132 de la base en faisant glisser les nervures latérales 231, 232 dans les rainures ou glissières 133, 134 de la base. Le pignon 330 vient alors au contact de la crémaillère 135 pour s'y engager. Le kératome est alors prêt à fonctionner.

On signalera la possibilité d'une variante non représentée qui consiste, dans le carter 304, en ce que l'arbre 301a soit prévu pour entraîner un pignon 330 décalé qui engrène avec un autre pignon identique dans le même plan. Le bras 132 est alors pourvu d'une crémaillère semblable à la crémaillère 135 qui lui fait face et chaque pignon s'engage sur la crémaillère correspondante. On crée ainsi une propulsion de la tête entre les bras 131, 132, au-dessus de l'anneau 101 au moyen de deux forces au lieu d'une afin d'éviter un éventuel coincement de la tête dans ses glissières qui pourrait provenir du couple auquel est soumis la tête dans le cas d'un entraînement par un seul pignon et une seule crémaillère.

On notera que l'indépendance des motorisations offre de nombreux avantages au chirurgien quant à la souplesse d'emploi du microkératome. Outre ceux déjà mentionnés, on indiquera qu'il est confortable pour le chirurgien de lancer le mouvement d'oscillation de la lame avant le mouvement de coupe de la tête de coupe, et qu'il peut être intéressant de conserver le mouvement d'oscillation de la lame alors même que le mouvement de la tête de coupe s'arrête.

Dans le cas du kératome pivotant, cette double motorisation présente également l'avantage de prévoir une fabrication standard de deux types de microkératomes, soit à avance motorisée, soit à avance manuelle. En effet, pour un kératome à avance manuelle, la seule modification mineure à apporter à celui qui est décrit réside dans l'absence de mortaise 107 au sommet du pivot 106. Dans ce cas, le chirurgien peut actionner manuellement l'ensemble des éléments 200, 300 autour du pivot 106 après les avoir verrouillés sur ce pivot. Il perd cependant l'avantage de disposer d'une position de départ déterminée et fixe. Pour conserver cet avantage, une modification simple du kératome selon l'invention consiste à supprimer le moteur 303 et à le remplacer par une pièce qui présente, comme l'arbre 306, un tenon d'extrémité. Cette pièce est soumise à un couple de rappel relativement faible autour de son axe longitudinal qui plaque le tenon contre la surface 309 de la pièce 301a comme précédemment décrit. Dans ce cas, le montage et l'assemblage des trois parties du kératome se réalisent de la même manière que celle décrite ci-dessus, le mouvement d'avance manuelle de la tête de coupe au-dessus de l'anneau de la base annulaire étant réalisé manuellement à l'encontre de l'organe exerçant le couple de rappel susdit sur le tenon. On indiquera enfin que cette version manuelle peut aisément être motorisée. Il suffira alors de procéder à la substitution inverse de celle décrite, c'est-à-dire la substitution du pion à tenon avec couple de rappel par un groupe moteur tel que celui 303 décrit ci-dessus.

## Revendications

1. Dispositif de chirurgie cornéenne comprenant :
- une base annulaire (100) de fixation de l'appareil sur l'oeil d'un patient,
- une tête de coupe (200) comportant un corps (200a) et une lame (201) et susceptible d'être déplacée selon une trajectoire plane parallèle à la base annulaire (100),
- des moyens de guidage (106) de la tête de coupe (200) par rapport à la base (100),
- des moyens d'entraînement (302) de la lame (201) dans la tête de coupe (200) selon un mouvement linéaire alternatif parallèle à son fil de coupe (201b),
- des moyens d'entraînement (303) de la tête de coupe (200) par rapport à la base annulaire (100) selon la trajectoire susdite comprenant deux ensembles moteurs indépendants, surmontant la tête de coupe
**caractérisé en ce que** lesdits moyens d'entraînement (302, 303) possèdent des arbres de sortie (306, 310) parallèles entre eux et perpendiculaires au plan de la trajectoire de la tête de coupe (200).

2. Dispositif selon la revendication 1, **caractérisé en ce que** les deux ensembles moteurs (302, 303) sont logés dans un carter (301) commun possédant une surface d'extrémité de laquelle fait saillie un arbre d'entraînement (310) de la lame de coupe (201), le carter comportant des moyens d'accouplement (312) de la tête de coupe (200).

3. Dispositif selon la revendication 2, **caractérisé en ce que** les moyens d'accouplement (312) de la tête de coupe (200) comprennent des moyens de liaison à baïonnette de cette tête au carter pour immobiliser le corps (200a) de la tête et le carter (301) le long de l'axe de l'arbre d'entraînement (310) de la lame (201) et des moyens de butée (212, 230, 301a, 320) entre le carter (301) et le corps (200a) de la tête pour indexer ce dernier et le carter dans une position angulaire déterminée autour de l'axe de l'arbre d'entraînement (310) de la lame (201).

4. Dispositif selon la revendication 3, **caractérisé en ce que** les moyens d'accouplement à baïonnette comportent un manchon (312) solidaire du carter (301) entourant la partie saillante de l'arbre d'entraînement (310) de la lame et un évidement (208, 209) de forme correspondante dans le corps (200a) de la tête de coupe (200).

5. Dispositif selon la revendication 3, **caractérisé en ce que** les moyens d'accouplement à baïonnette comportent un évidement coaxial à l'arbre d'entraînement (310) de la lame ménagé à l'extrémité du carter (301) et un manchon (210) en saillie du corps (200a) de la tête de coupe (200), prévu pour.coopérer par l'extérieur avec ledit évidement et pour recevoir à l'intérieur l'arbre d'entraînement (310) de la lame.

6. Dispositif selon l'une des revendications 2 à 5, **caractérisé en ce que** l'ensemble moteur (303) d'entraînement de la tête de coupe (200) par rapport à la base annulaire (100) comporte un arbre (306) dont l'extrémité est située au voisinage de la surface d'extrémité du carter (301) et possède des reliefs (307) ménagés en bout d'arbre pour coopérer par emboîtement axial avec des reliefs (107) ménagés à l'extrémité d'un pivot fixe (106) porté par la base annulaire (100) et parallèle aux axes des ensembles moteurs (302, 303).

7. Dispositif selon la revendication 6, **caractérisé en ce que** le corps (200a) de la tête de coupe (200) possède un évidement cylindrique (204) traversant la totalité de son épaisseur et coaxial à l'arbre (306) pourvu de reliefs d'extrémité (307) lorsque le corps (200a) de la tête de coupe est angulairement indexé par rapport au carter (301).

8. Dispositif selon la revendication 6 ou la revendication 7, **caractérisé en ce que** l'arbre (306) à reliefs d'extrémité et le carter (301) possèdent des moyens de butée (307, 309) définissant une position angulaire déterminée de l'arbre (306) à reliefs d'extrémité par rapport au carter (301).

9. Dispositif selon la revendication 7, **caractérisé en ce qu'**il comporte, entre le pivot (106) et le carter (301) ou le corps (200a) de la tête de coupe (200), des moyens (108, 111, 214, 318) de liaison axiale du pivot (106) et de l'arbre (306) à reliefs d'extrémité (307) pendant au moins le mouvement de la tête de coupe autour du pivot.

10. Dispositif selon la revendication 9, **caractérisé en ce que** les moyens de liaison axiale comportent un organe de commande de leur état activé/désactivé formé par une bague (315) montée tournante sur le carter (301) autour d'un axe parallèle aux axes des moteurs (302, 303).

11. Dispositif selon la revendication 8, **caractérisé en ce que** la position angulaire déterminée entre l'arbre (306) à reliefs d'extrémité (307) et le carter (301) détermine la position de début de la trajectoire de la tête de coupe (200) lors de la liaison en rotation de l'arbre (306) avec le pivot (106).

12. Dispositif selon la revendication 6, **caractérisé en ce que** la base annulaire (100) comporte, dans une zone sensiblement diamétralement opposée au pivot fixe (106), une butée (113) de limitation du pivotement du corps (200a) de la tête de coupe (200) par rapport à la base (100), pour coopérer avec une projection radiale (211) de ce corps (200a) de la tête de coupe.

13. Dispositif selon la revendication 12, **caractérisé en ce que** la butée (113) et/ou la projection radiale (211) sont à position réglable sur la base annulaire (100) et/ou sur le corps (200a) de la tête de coupe (200).

14. Dispositif selon l'une des revendications 1 à 4, **caractérisé en ce que** la base annulaire (100) comporte deux bras supérieurs (131, 132) placés à l'arrière de l'axe (X) de l'anneau (101) et sensiblement parallèles à cet axe, formant un préhenseur de la base.

15. Dispositif selon la revendication 14, **caractérisé en ce que** la racine de liaison des bras (131, 132) à l'anneau (101) comporte deux glissières (133, 134) parallèles au plan supérieur de l'anneau (101).

16. Dispositif selon la revendication 15, **caractérisé en ce que** la racine de liaison à l'anneau (101) d'au moins l'un des bras (131) comporte sur sa face interne une butée (137) réglable en position dans la direction des glissières (133, 134).

17. Dispositif selon la revendication 16, **caractérisé en ce que** le carter (301) comporte un doigt latéral (331) pour coopérer avec la butée (137) de la base.

18. Dispositif selon l'une des revendications 15 à 17, **caractérisé en ce que** la face interne d'au moins l'une des racines de bras (131) porte une crémaillère rectiligne (135).

19. Dispositif selon la revendication 18, **caractérisé en ce que** l'ensemble moteur (303) d'entraînement de la tête de coupe (200) par rapport à la base annulaire (100) comporte un arbre (304) de sortie accouplé en rotation à un pignon d'extrémité (330) qui fait saillie latéralement du carter (301) et dont l'extrémité axiale est située sensiblement dans le plan d'extrémité de ce carter (301).

20. Dispositif selon l'une des revendications 15 à 19, **caractérisé en ce que** le corps (200a) de la tête de coupe (200) possède des coulisses latérales (231, 232) pour coopérer avec les glissières (133, 134) de la base 100.

21. Dispositif selon l'une des revendications précédentes, **caractérisé en ce qu'**il comprend un jeu de têtes de coupe dans lequel chaque tête de coupe comporte un plateau aplanateur (205) distant d'une surface (220, 221) de glissement de la tête sur l'embase d'une valeur D différente de celle des autres têtes du jeu.

22. Dispositif selon l'une des revendications précédentes, **caractérisé en ce que** la tête de coupe (200) comporte un logement (206) de guidage de la lame de coupe (201) dans son mouvement linéaire alternatif, lequel logement possède des moyens (206a) de maintien de la lame (201) à l'intérieur du logement.

## Patentansprüche

1. Vorrichtung für die Hornhautchirurgie, umfassend:
- eine ringförmige Basis (100) zur Befestigung des Gerätes am Auge eines Patienten,
- einen Schneidkopf (200), der einen Körper (200a) und eine Schneidklinge (201) umfasst und dazu geeignet ist, entlang einer ebenen Bahn, die parallel zur ringförmigen Basis (100) ist, verschoben zu werden,
- Führungsmittel (106) zum Führen des Schneidkopfes (200) relativ zur Basis (100),
- Antriebsmittel (302) zum Antrieb der Schneidklinge (201) in dem Schneidkopf (200) gemäß einer linearen Hin- und Herbewegung parallel zu ihrer Schneide (201b),
- Antriebsmittel (303) zum Antrieb des Schneidkopfes (200) relativ zur ringförmigen Basis (100) entlang der oben genannten Bahn, die zwei unabhängige Motoreinheiten umfassen, die über dem Schneidkopf angeordnet sind,
**dadurch gekennzeichnet, dass** die genannten Antriebsmittel (302, 303) Abtriebswellen (306, 310) besitzen, die parallel zueinander und senkrecht zur Ebene der Bahn des Schneidkopfes (200) verlaufen.

2. Vorrichtung nach Anspruch 1, **dadurch gekennzeichnet, dass** die zwei Motoreinheiten (302, 303) in einem gemeinsamen Gehäuse (301) untergebracht sind, das eine Stirnfläche aufweist, von der eine Antriebswelle (310) zum Antrieb der Schneidklinge (201) absteht, wobei das Gehäuse Kopplungsmittel (312) zum Ankoppeln des Schneidkopfes (200) umfasst.

3. Vorrichtung nach Anspruch 2, **dadurch gekennzeichnet, dass** die Kopplungsmittel (312) zum Ankoppeln des Schneidkopfes (200) Bajonettverbindungsmittel zur Verbindung dieses Kopfes mit dem Gehäuse umfassen, um den Körper (200a) des Kopfes und das Gehäuse (301) entlang der Achse der Antriebswelle (310) für die Schneidklinge (201) zu blockieren, sowie Anschlagsmittel (212, 230, 301a, 320) zwischen dem Gehäuse (301) und dem Körper (200a) des Kopfes um diesen letztgenannten und das Gehäuse in einer bestimmten Winkelposition um die Achse der Antriebswelle (310) für die Schneidklinge (201) zu indexieren.

4. Vorrichtung nach Anspruch 3, **dadurch gekennzeichnet, dass** die Bajonettverbindungsmittel eine fest mit dem Gehäuse (301) verbundene Hülse (312) umfassen, die den vorstehenden Teil der Antriebswelle (310) für die Schneidklinge umgibt, sowie eine Aussparung (208, 209) mit entsprechender Form in dem Körper (200a) des Schneidkopfes (200).

5. Vorrichtung nach Anspruch 3, **dadurch gekennzeichnet, dass** die Bajonettverbindungsmittel eine Aussparung umfassen, die koaxial zur Antriebswelle (310) für die Schneidklinge und am Ende des Gehäuses (301) ausgebildet ist, sowie eine vom Körper (200a) des Schneidkopfes (200) abstehende Hülse (210), die vorgesehen ist, um mit ihrem Äußeren mit der genannten Aussparung zusammenzuwirken und in ihrem Inneren die Antriebswelle (310) für die Schneidklinge aufzunehmen.

6. Vorrichtung nach einem der Ansprüche 2 bis 5, **dadurch gekennzeichnet, dass** die Antriebsmotoreinheit (303) zum Antrieb des Schneidkopfes (200) relativ zur ringförmigen Basis (100) eine Welle (306) umfasst, deren Ende sich nahe der Stirnfläche des Gehäuses (301) befindet und die Reliefelemente (307) aufweist, die am Wellenende ausgebildet sind, um durch axiales Ineinanderstecken mit Reliefelementen (107) zusammenzuwirken, die am Ende eines ortsfesten Drehzapfens (106) ausgebildet sind, der von der ringförmigen Basis (100) getragen wird und parallel zu den Achsen der Motoreinheiten (302, 303) verläuft.

7. Vorrichtung nach Anspruch 6, **dadurch gekennzeichnet, dass** der Körper (200a) des Schneidkopfes (200) eine zylindrische Aussparung (204) besitzt, die seine gesamte Dicke durchsetzt und koaxial zu der mit Endreliefelementen (307) versehenen Welle (306) ist, wenn der Körper (200a) des Schneidkopfes relativ zu dem Gehäuse (301) in einer Winkelstellung indexiert ist.

8. Vorrichtung nach Anspruch 6 oder Anspruch 7, **dadurch gekennzeichnet**, das die mit Endreliefelementen versehene Welle (306) und das Gehäuse (301) Anschlagmittel (307, 309) besitzen, die eine bestimmte Winkelposition der mit Endreliefelementen versehenen Welle (306) relativ zum Gehäuse (301) festlegen.

9. Vorrichtung nach Anspruch 7, **dadurch gekennzeichnet, dass** sie zwischen dem Drehzapfen (106) und dem Gehäuse (301) oder dem Körper (200a) des Schneidkopfes (200) Mittel (108, 111, 214, 318) zur axialen Verbindung des Drehzapfens (106) mit der mit Endreliefelementen (307) versehenen Welle (306) wenigstens während der Bewegung des Schneidkopfes um den Drehzapfen umfasst.

10. Vorrichtung nach Anspruch 9, **dadurch gekennzeichnet, dass** die Mittel zur axialen Verbindung ein Steuerelement zur Steuerung ihres aktiven/inaktiven Zustandes umfassen, das aus einem Ring (315) gebildet ist, der am Gehäuse (301) drehbar um eine Achse gelagert ist, die parallel zu den Achsen der Motoren (302, 303) verläuft.

11. Vorrichtung nach Anspruch 8, **dadurch gekennzeichnet, dass** die vorgegebene Winkelposition zwischen der mit Endreliefelementen (307) versehenen Welle (306) und dem Gehäuse (301) die Anfangsposition der Bahn des Schneidkopfes (200) während der Rotationsverbindung der Welle (306) mit dem Drehzapfen (106) bestimmt.

12. Vorrichtung nach Anspruch 6, **dadurch gekennzeichnet, dass** die ringförmige Basis (100) in einem dem ortsfesten Drehzapfen (106) im wesentlichen diametral gegenüberliegenden Bereich einen Anschlag (113) zur Begrenzung der Schwenkbewegung des Körpers (200a) des Schneidkopfes (200) relativ zur Basis (100) umfasst, um mit einem radialen Vorsprung (211) dieses Körpers (200a) des Schneidkopfes zusammenzuwirken.

13. Vorrichtung nach Anspruch 12, **dadurch gekennzeichnet, dass** der Anschlag (113) und/oder der radiale Vorsprung (211) an der ringförmigen Basis (100) und/oder an dem Körper (200a) des Schneidkopfes (200) verstellbar sind.

14. Vorrichtung nach einem der Ansprüche 1 bis 4, **dadurch gekennzeichnet, dass** die ringförmige Basis (100) zwei obere Arme (131, 132) umfasst, die rückwärts der Achse (X) des Ringes (101) im wesentlichen parallel zu dieser Achse angeordnet sind und einen Griff für die Basis bilden.

15. Vorrichtung nach Anspruch 14, **dadurch gekennzeichnet, dass** die Ansatzstelle der Arme (131, 132) an dem Ring (101) zwei Gleitschienen (133, 134) umfasst, die parallel zur oberen Ebene des Ringes (101) verlaufen.

16. Vorrichtung nach Anspruch 15, **dadurch gekennzeichnet, dass** die Ansatzstelle mindestens eines der Arme (131) an dem Ring (101) auf ihrer Innenseite einen Anschlag (137) umfasst, der in Richtung der Gleitschienen (133, 134) in seiner Position verstellt werden kann.

17. Vorrichtung nach Anspruch 16, **dadurch gekennzeichnet, dass** das Gehäuse (301) einen seitlichen Zapfen (331) zum Zusammenwirken mit dem Anschlag (137) der Basis umfasst.

18. Vorrichtung nach einem der Ansprüche 15 bis 17, **dadurch gekennzeichnet, dass** die Innenseite mindestens einer der Ansatzstellen der Arme (131) eine geradlinige Zahnstange (135) trägt.

19. Vorrichtung nach Anspruch 18, **dadurch gekennzeichnet, dass** die Antriebsmotoreinheit (303) zum Antrieb des Schneidkopfes (200) relativ zur ringförmigen Basis (100) eine Abtriebswelle (304) umfasst, die drehfest an einen Stirnzapfen (330) gekoppelt ist, der seitlich von dem Gehäuse (301) absteht und dessen axiales Ende sich im wesentlichen in der Stirnebene dieses Gehäuses (301) befindet.

20. Vorrichtung nach einem der Ansprüche 15 bis 19, **dadurch gekennzeichnet, dass** der Körper (200a) des Schneidkopfes (200) seitliche Kulissen (231, 232) zum Zusammenwirken mit den Gleitschienen (133, 134) der Basis (100) aufweist.

21. Vorrichtung nach einem der vorhergehenden Ansprüche, **dadurch gekennzeichnet, dass** sie einen Satz von Schneidköpfen umfasst, bei dem jeder Schneidkopf eine Planierungsplatte (205) umfasst, die zu einer zum Gleiten auf der Basis dienenden Gleitfläche (220, 221) des Kopfes um einen Wert D beabstandet ist, der von dem der anderen Köpfe des Satzes abweicht.

22. Vorrichtung nach einem der vorhergehenden Ansprüche, **dadurch gekennzeichnet, dass** der Schneidkopf (200) eine Aufnahme (206) zur Führung der Schneidklinge (201) in ihrer linearen Hin- und Herbewegung umfasst, wobei die Aufnahme Mittel (206a) zum Halten der Schneidklinge (201) im Inneren der Aufnahme besitzt.

## Claims

1. A device for surgery of the cornea, the device comprising:
· an annular base (100) for fixing the apparatus to the eye of a patient;
· a cutter head (200) comprising a body (200a) and a blade (201) suitable for being displaced along a plane trajectory parallel to the annular base (100);
· guide means (106) for guiding the cutter head (200) relative to the base (100);
· drive means (302) for driving the blade (201) in the cutter head (200) with reciprocating linear motion parallel to its cutting edge (201b); and
· drive means (303) for driving the cutter head (200) relative to the annular base (100) along said trajectory, which drive means comprise two independent motor units surmounting the cutter head;
the device being **characterized in that** said drive means (302, 303) have mutually parallel outlet shafts (306, 310) perpendicular to the plane of the trajectory of the cutter head (200).

2. A device according to claim 1, **characterized in that** the two motor units (302, 303) are received in a common case (301) possessing an end surface from which there projects a drive shaft (310) for driving the cutter blade (201), the case having coupling means (312) for coupling with the cutter head (200).

3. A device according to claim 2, **characterized in that** the coupling means (312) for coupling to the cutter head (200) comprise bayonet means for connecting said head to the case so as to prevent the body (200a) of the head and the case (301) from moving relative to each other along the axis of the drive shaft (310) for driving the blade (201), and abutment means (212, 230, 301a, 320) between the case (301) and the body (200a) of the head to index the body and the case in a determined angular position about the axis of the drive shaft (310) for driving the blade (201).

4. A device according to claim 3, **characterized in that** the bayonet coupling means comprise a sleeve (312) secured to the case (301) surrounding the projecting portion of the drive shaft (310) for driving the blade and a recess (208, 209) of complementary shape in the body (200a) of the cutter head (200).

5. A device according to claim 3, **characterized in that** the bayonet coupling means comprise a recess on the axis of the blade drive shaft (310) formed in the end of the case (301) and a sleeve (210) projecting from the body (200a) of the cutter head (200), for co-operating on the outside with said recess and for receiving on the inside the blade drive shaft (310).

6. A device according to any one of claims 2 to 5, **characterized in that** the drive motor unit (303) for driving the cutter head (200) relative to the annular base (100) has a shaft (306) whose end is situated in the vicinity of the end surface of the case (301) and possess portions in relief (307) arranged at the end of the shaft to co-operate by axial engagement with portions in relief (107) arranged at the end of a fixed pivot (106) carried by the annular base (100) and parallel to the axes of the motor units (302, 303).

7. A device according to claim 6, **characterized in that** the body (200a) of the cutter head (200) possess a cylindrical recess (204) passing right through its thickness and on the same axis as the shaft (306) which is provided with end portions in relief (307) when the body (200a) of the cutter head is angularly indexed relative to the case (301).

8. A device according to claim 6 or claim 7, **characterized in that** the shaft (306) having end portions in relief and the case (301) possess abutment means (307, 309) defining a determined angular position relative to the case (301) for the shaft (306) having end portions in relief.

9. A device according to claim 7, **characterized in that** it includes, between the pivot (106) and the case (301) or the body (200a) of the cutter head (200), means (108, 111, 214, 318) for axially connecting the pivot (106) and the shaft (306) having end portions in relief (307) at least during the movement of the cutter head about the pivot.

10. A device according to claim 9, **characterized in that** the axial connection means include a member for controlling their activated and deactivated states, the member being in the form of a ring (315) mounted to turn on the case (301) about an axis parallel to the axes of the motors (302, 303).

11. A device according to claim 8, **characterized in that** the determined angular position between the shaft (306) having end portions in relief (307) and the case (301) determines the starting position for the trajectory of the cutter head (200) when making the rotary connection between the shaft (306) and the pivot (106).

12. A device according to claim 6, **characterized in that** the annular base (100) includes, in a zone that is substantially diametrically opposite the fixed pivot (106), an abutment (113) for limiting the pivoting of the body (200a) of the cutter head (200) relative to the base (100), for co-operating with a radial projection (211) of said body (200a) of the cutter head.

13. A device according to claim 12, **characterized in that** the abutment (113) and/or the radial projection (211) are adjustable in position on the annular base (100) and/or on the body (200a) of the cutter head (200).

14. A device according to any one of claims 1 to 4, **characterized in that** the annular base (100) includes two upper arms (131, 132) placed behind the axis (X) of the ring (101) and substantially parallel to the axis, forming a handle for the base.

15. A device according to claim 14, **characterized in that** the roots connecting the arms (131, 132) to the ring (101) include two slideways (133, 134) that are parallel to the top plane of the ring (101).

16. A device according to claim 15, **characterized in that** the root for connecting at least one of the arms (131) to the ring (101) includes, on its inside face, an abutment (137) whose position can be adjusted in the direction of the slideways (133, 134).

17. A device according to claim 16, **characterized in that** the case (301) includes a lateral finger (331) for co-operating with the abutment (137) of the base.

18. A device according to any one of claims 15 to 17, **characterized in that** the inside face of at least one of the arm roots (131) carries a rectilinear rack (135).

19. A device according to claim 18, **characterized in that** the motor assembly (303) for driving the cutter head (200) relative to the annular base (100) includes an outlet shaft (304) coupled in rotation to an end gearwheel (330) which projects laterally from the case (301) and having an axial end that is situated substantially in the end plane of the case (301).

20. A device according to any one of claims 15 to 19, **characterized in that** the body (200a) of the cutter head (200) has lateral sliding pieces (231, 232) for co-operating with the slideways (133, 134) of the base (100).

21. A device according to any preceding claim, **characterized in that** it comprises a set of cutter heads in which each cutter head includes a flattening shelf (205) spaced apart from a sliding surface (220, 221) of the head on the base by a distance D that is different from said distance for all of the other heads of the set.

22. A device according to any preceding claim, **characterized in that** the cutter head (200) has a housing (206) for guiding the cutter blade (201) in its reciprocating linear motion, which housing possesses means (206a) for holding the blade (201) inside the housing.
